(19) Europäisches Patentamt
European Patent Office
Office européen des brevets

(11) **EP 3 854 405 A1**

(12) **EUROPEAN PATENT APPLICATION**
published in accordance with Art. 153(4) EPC

(43) Date of publication:
**28.07.2021 Bulletin 2021/30**

(51) Int Cl.:
**A61K 31/7088** (2006.01)   **A61K 38/20** (2006.01)
**A61P 35/00** (2006.01)

(21) Application number: **19862297.9**

(22) Date of filing: **19.09.2019**

(86) International application number:
**PCT/KR2019/012178**

(87) International publication number:
**WO 2020/060247 (26.03.2020 Gazette 2020/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.09.2018 KR 20180112506**

(71) Applicant: **Autotelic Bio Inc.**
**Cheongju-si, Chungcheongbuk-do 28160 (KR)**

(72) Inventors:
• **KIM, Tae Hun**
  **Yeonseo-myeon Sejong 30043 (KR)**
• **PARK, Jun Eui**
  **Seongnam-si Gyeonggi-do 13623 (KR)**
• **LEE, Jeong Min**
  **Seoul 05503 (KR)**

(74) Representative: **Louis Pöhlau Lohrentz**
**Patentanwälte**
**Postfach 30 55**
**90014 Nürnberg (DE)**

(54) **ANTICANCER COMPOSITION**

(57) Disclosed is an anticancer formula composition comprising trabedersen and IL-2.

【Fig. 9】

EP 3 854 405 A1

**Description**

[Technical Field]

The present invention relates to a composition of combination for treating cancer. More specifically, the present invention relates to a composition of combination for treating cancer comprising trabedersen and IL-2 (interleukin-2), and an administration of combination thereof.

[Background Art]

The existing cytotoxic anticancer drugs have a problem that it attacks not only cancer cells but also normal cells. Therefore, targeted anticancer drugs have become the main stream for the development of anticancer drugs in order to solve such problems. Targeted anticancer drugs attack only specific target factors selectively while they do not attack normal cells during the carcinogenesis process. Therefore, the pains caused by the targeted anticancer drugs to patients are less than that of existing cytotoxic anticancer drugs.

However, these targeted anticancer agents were also found to have a disadvantage in that a resistance occurred after administration. In order to overcome the shortcomings of these targeted anticancer drugs and prolong the survival period of patients through anticancer treatment, immunotherapeutic anticancer drugs are emerging as the core of the development of anticancer drugs.

The present invention is to provide an anticancer agent with improved anticancer effect while reducing adverse effects during treatment and an administration of combination thereof.

[Prior Document]

[Non-patent Document]

(Non-patent Document 1) Schlingensiepen KH et al, Cancer Sci. 2011; 102 : 1193-200

[Disclosure]

[Technical Problem]

The present invention is to enhance the anticancer effect by co-administration of TGF-$\beta$2 inhibitor, Trabedersen, and IL-2.

[Technical Solution]

In order to solve the above problems, the present invention provides a composition of combination for treating cancer comprising a TGF-$\beta$ inhibitor and a cytokine.

Specifically, the present invention provides a composition of combination for treating cancer comprising trabedersen which is a TGF-$\beta$ inhibitors and interleukin (IL)-2.

Expression of TGF-$\beta$ in tumors performs a comprehensive immune suppression function. TGF-$\beta$ is a biomarker that shows a resistance to immunotherapeutic anticancer drugs.

Immune checkpoints such as PD-1 and PD-L1 play a role in part in comprehensive immune suppression. Therefore, if an inhibitor against the immune checkpoints such as PD-1 and PD-L1 is used as immunotherapeutic anticancer drugs in a condition without removing TGF-$\beta$ in order to restore immune function, it works only partially, and cell death function of the immune system cannot be completely restored.

Trabedersen is a synthetic 18-mer phosphorothioate antisense oligonucleotide specifically designed for targeting human TGF-$\beta$, and developed based on data showing the correlation between TGF-$\beta$ overexpression and tumor progression. The correlation between TGF-$\beta$ overexpression and tumor progression has been evaluated in a number of in-vitro and in-vivo non-clinical models (Schlingensiepen KH et al, Cancer Sci. 2011; 102: 1193-200).

IL-2 (interleukin-2), a kind of cytokine, stimulates the immune system to increase the size of immune cells extensively.

[Advantageous Effect]

The composition of combination for treating cancer according to the present invention synergistically enhances the anticancer effect while reducing adverse effects when administered.

[Description of Drawings]

Fig. 1 shows the results of relative qualitative analysis of TGF-β1 mRNA and TGF-β2 mRNA for various cancer cell lines.
Fig. 2 shows the results of quantitative analysis of TGF-β1 mRNA from various cancer cell lines.
Fig. 3 shows the results of quantitative analysis of TGF-β2 mRNA from various cancer cell lines.
Figs. 4 to 8 show the results of growth inhibition analysis in cell lines of breast cancer, pancreatic cancer, melanoma, lung cancer and colorectal cancer with treatment of trabedersen alone using EZCYTOX.
Figs. 9 to 13 show the cell death effect of combination of human PBMC activated by IL-2 and trabedersen.
Figs. 14 and 15 show the in vivo anticancer effect of combination of IL-2 and trabedersen in immunodeficient mice (NSG mice) humanized with human PBMC.

[Mode for Invention]

The present invention relates to a composition of combination for treating cancer comprising trabedersen and IL-2. The composition according to the invention is particularly useful for the treatment of solid cancers (for example, breast cancer, pancreatic cancer, melanoma, lung cancer or colorectal cancer, etc.).

When administering the composition of the present invention, trabedersen and IL-2 may be administered in combination in a manner of administration sequentially or simultaneously, or in a manner of administration separately. Therefore, when administering the composition of the present invention, each ingredient is simultaneously present in a therapeutically effective amount in vivo to exhibit a synergistic effect with each other.

In other aspect of the present invention, the present invention relates to a composition for treating cancer comprising trabedersen for administration in combination with IL-2. Such a composition according to the present invention is particularly useful for the treatment of solid cancers (for example, breast cancer, pancreatic cancer, melanoma, lung cancer or colorectal cancer, etc.).

In another aspect of the present invention, the present invention relates to a composition for treating cancer composition IL-2 for administration in combination with trabedersen. The composition according to the invention is particularly useful for the treatment of solid cancers (for example, breast cancer, pancreatic cancer, melanoma, lung cancer or colorectal cancer, etc.).

Hereinafter, the present invention will be described in detail with reference to examples. However, the following examples are only to illustrate the present invention, and the spirit or scope of the present invention is not limited thereto.

In order to verify the anticancer effect of the composition according to the present invention, an in-vitro experiment for anticancer effect was conducted in a mouse of solid cancer model by administering trabedersen which is a TGF-β2 inhibitor and IL-2 in combination. For example, the amount of TGF-β mRNA expression in various solid cancer cell lines was checked, and in these cells, cell death was verified in cancer cells which are treated with trabedersen alone or treated with trabedersen in combination with PBMC (peripheral blood mononuclear cells) activated by IL-2. In addition, the effect of inhibiting the growth of human cancer cells (xenograft model) was verified in immunodeficient mice humanized with human PBMC by administering trabedersen and IL-2 in combination.

**Experimental Example 1: Quantitative and relative qualitative analysis of TGF-β2 mRNA levels in various carcinomas**

1) TGF-β2 mRNA expression analysis

The experiment was performed using the cell lines shown in Table 1 below.

[Table 1]

| Tumor cells | tissue | Diagnosis | Used culture medium | Purchase |
|---|---|---|---|---|
| MDA-MB-231 | Breast | Adenocarcinoma | RPMI 1640 (10% FBS, 1% Antibiotics, HEPES) | |
| Hs578T | | carcinoma | DMEM (10% FBS, 1% Antibiotics) | |
| BT 549 | Breast | ductal carcinoma | DMEM (10% FBS, 1% Antibiotics) | |
| AsPC-1 | Pancreas | adenocarcinoma | RPMI 1640 (10% FBS, 1% Antibiotics, HEPES) | ATCC |
| Capan-2 | | | | |

(continued)

| Tumor cells | tissue | Diagnosis | Used culture medium | Purchase |
|---|---|---|---|---|
| SK-MEL-28 | Skin | malignant melanoma | DMEM (10% FBS, 1% Antibiotics) | |
| Hs294T | | Melanoma | | |
| A549 | Lung | carcinoma | RPMI 1640 (10% FBS, 1% Antibiotics, HEPES) | |
| HT29 DLD-1 | Colon | adenocarcinoma | RPMI 1640 (10% FBS, 1% Antibiotics, HEPES) | |
| LS174T | | | DMEM (10% FBS, 1% Antibiotics) | |

RNA was prepared from each of $1\times10^7$ cells lines in Table 1 using an RNA prep kit (Qiagen), and then cDNA was synthesized at 37°C for 1 hour using 1 $\mu$g of purified RNA as a template.

PCR was performed on 10 $\mu\ell$ of cDNA using TGF-$\beta$2 specific primers F (ATCGATGGCACCTCCACATATG) and R (GCGAAGGCAGCAATTATGCTG) (Denaturation 95°C 1 min, Annealing 57°C 1 min, Extension 72°C 1 min, 35 cycles and Final Extension 72°C 5min). Then, mRNA expression of TGF-$\beta$1 and TGF-$\beta$2 was verified using the band intensity of TGF-$\beta$1 and TGF-$\beta$2 by comparing with the PCR results for GAPDH (Glyceraldehyde 3-phosphate dehydrogenase).

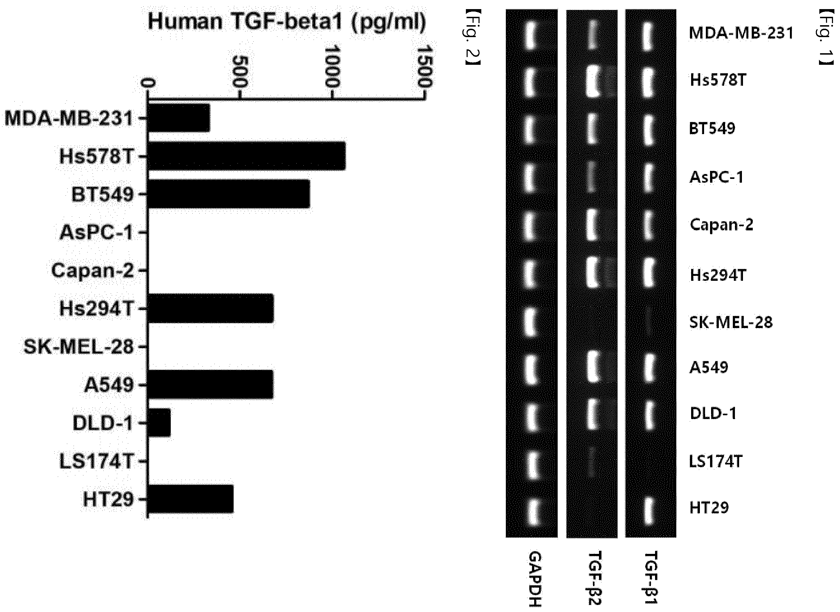

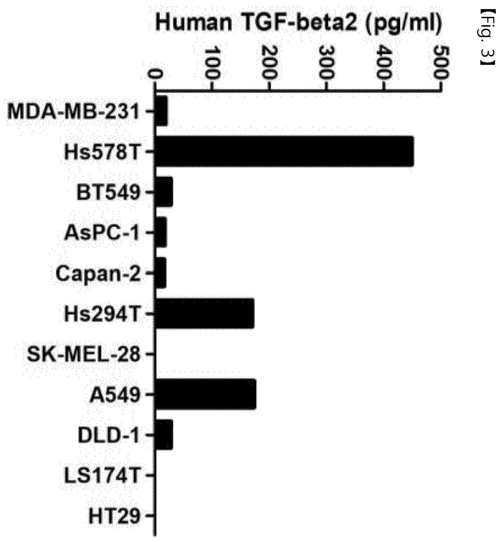

Figs. 1 to 3 show results of relative qualitative analysis and quantitative analysis for TGF-$\beta$1 or TGF-$\beta$2 mRNA.

As a result of the experiment, it can be seen that TGF-$\beta$ mRNA is relatively largely expressed in skin cancer cells, lung cancer cells, and breast cancer cells.

**Experimental Example 2: Cell death effect by treatment with Trabedersen alone in various solid cancer cell lines**

In various solid cancer cell lines, cell death effect was confirmed by treatment with Trabedersen alone.

1) Cell culture

As shown in Table 1 above, media such as RPMI 1640 (10% FBS, Antibiotic-Antimycotic, HEPES) and DMEM (10% FBS, Antibiotic-Antimycotic) were used depending on the cell type, and the cells were cultured in an environment of 37°C and 5% $CO_2$.

2) Trabedersen transfection

Each cell line described in Table 1 is seeded as shown in Table 2 below, and then cultured for 24 hours at 37°C, 5% $CO_2$, and then transfected with trabedersen:
The specific transfection method is as follows:
Trabedersen is diluted in serum free media (culture media A: RPMI 1640 or DMEM) to be of a concentration of 20nM, 40nM, 80nM and 160nM, respectively, in the finally prepared culture media (i.e., culture media comprising culture media A, culture media B and cell culture media described below).

Lipofectamine 2000 is added to a serum-free culture media (culture media B: RPMI 1640 or DMEM) in a predetermined amount so that the experimental conditions shown in Table 2 below may be achieved.

Reaction is conducted in culture media A and culture media B at room temperature for 5 minutes, respectively. Then, 50 $\mu\ell$ of culture media A and 50 $\mu\ell$ of culture media B (1:1 volume ratio) were mixed on a 12 well plate basis, and the mixture is reacted again at room temperature for 20 minutes.

The culture media of the cells prepared in the step of "1) Cell culture" above was replaced with a new 10% FBS culture media, and the mixture of culture media A and B wherein the reaction is completed is added to the cell culture media.

[Table 2]

| PLATE | Seeding density (number of cells/well) | Lipofectamine 2000 | Total volume (culture media A + culture media B + cell culture media) |
|---|---|---|---|
| 6 well | 100,000~200,000 | 4 $\mu\ell$ | 2 m$\ell$ |
| 12 well | 50,000 | 2 $\mu\ell$ | 1 m$\ell$ |
| 24 well | 10,000~30,000 | 1 $\mu\ell$ | 500 $\mu\ell$ |

(continued)

| PLATE | Seeding density (number of cells/well) | Lipofectamine 2000 | Total volume (culture media A + culture media B + cell culture media) |
|---|---|---|---|
| 96 well | 5,000~20,000 | 0.5 $\mu\ell$ | 100 $\mu\ell$ |

3) Cytotoxicity Assay

For cytotoxicity analysis, cell activity was measured in O.D. 450nm value by using EZCYTOX (Daeil Lab Service Co., Ltd.) on the 2nd day after trabedersen transfection. Apoptosis/necrosis analysis was performed by using FACS (Fluorescence activated cell sorter) through Annexin V and PI staining.

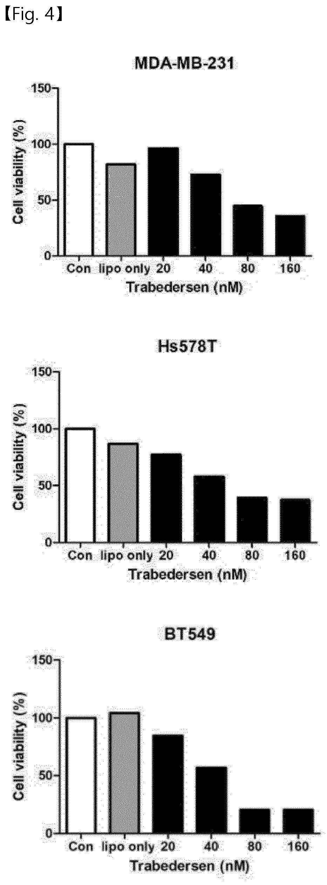

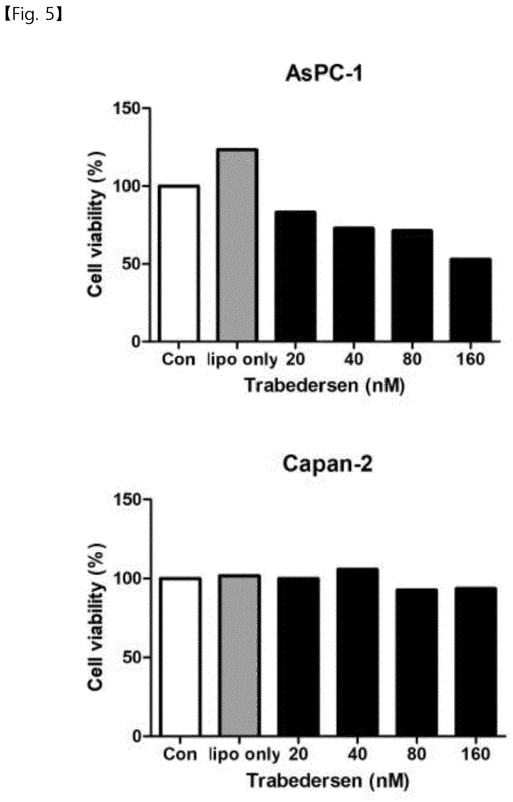

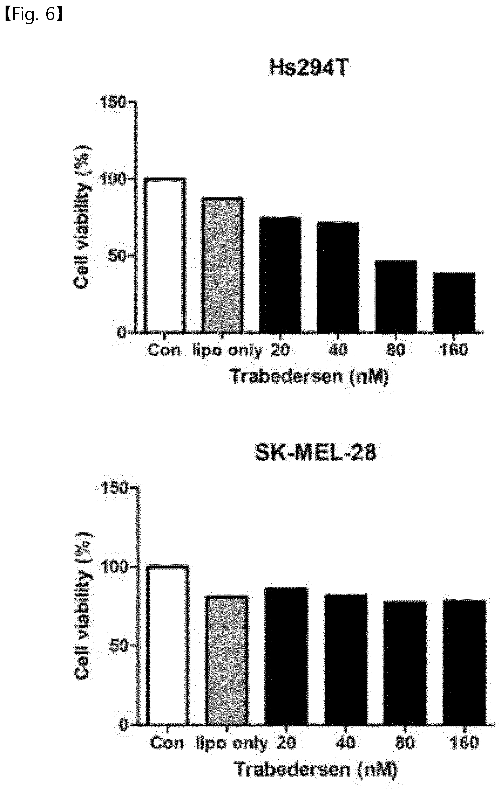

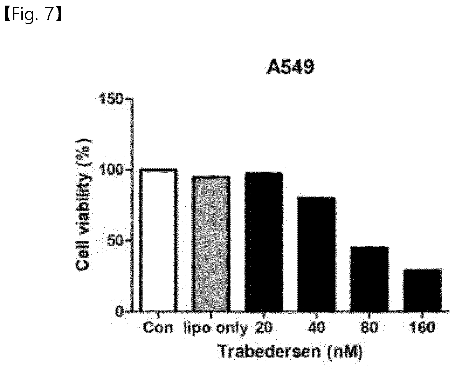

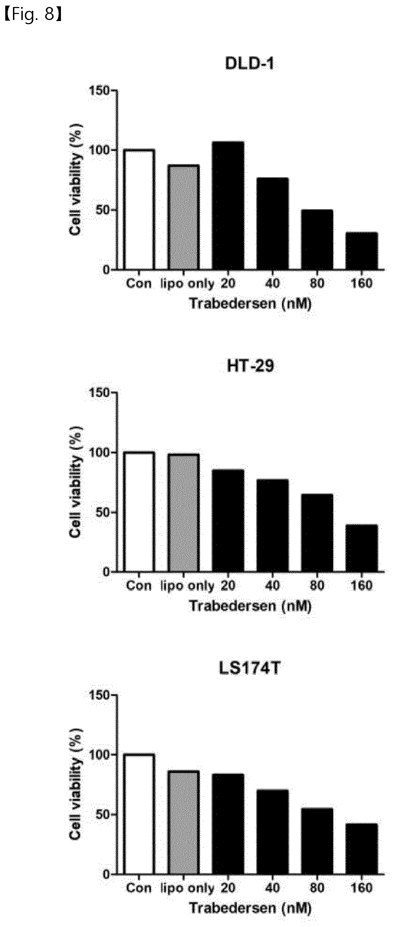

Figs. 4 to 8 are results of analysis for growth inhibition in cancer cell lines which are treated with trabedersen alone using EZCYTOX. It can be seen that the activity of the cancer cell line was inhibited by administration of trabedersen. However, it was observed that cell death effect was not so strong in Capan-2 and SK-MEL-28 cell lines.

**Experimental Example 3: Test of cell death effect in breast cancer cell line by co-administration of human PBMC activated with IL-2 and trabedersen in combination**

1) IL-2 activation

PBMCs (peripheral blood mononuclear cells) from peripheral blood were separated using ficoll-plaque plus (purchased from GE healthcare), and then cultured at a concentration of $4 \times 10^6/m\ell$ in culture media wherein 10% FBS and IL-2 (20ng/ml) were added. The expression of CD69 (T cell activation marker) in CD4+/CD8+ T cells was confirmed using FACS on the 5th day.

2) Analysis of co-administration of IL-2 and trabedersen in combination

As described above, PBMCs of peripheral blood were separated using ficoll-plaque plus (GE healthcare), and then cultured at a concentration of $4 \times 10^6/m\ell$ in culture media wherein 10% FBS and IL-2 (20ng/ml) were added. The cancer cells were transfected with trabedersen on the 3rd day of culture. After 24 hours, the culture media of the target cancer cells were replaced with a 10% FBS media. PBMC on the 5th day of IL-2 culture were added to 2m$\ell$ of 10% FBS RPMI media in a ratio of cancer cell lines (seeding) : Effector (cells with Immune effect) = 1 : 10 (the ratio of the number of cells), and then added on the plate and cultured.

Inactivated PBMCs were separated in isolation by ficoll and used as a control group. In addition, a cell lines without any PBMC were also used as another control group. During the step of transfection in the experiment, the concentrations of trabedersen in the culture media are set to be various (*i.e.,* each concentration of 0nM, 10nM and 40nM are tested).

After 24 hours, the result of cytotoxicity was analyzed through PI staining.

**Experimental Example 4: Test of cell death effect in pancreatic cancer cell line by co-administration of human PBMC activated with IL-2 and Trabedersen in combination**

Experiments were performed on the pancreatic cancer cell line (AsPC-1) in a similar manner to Experimental Example 3.

**Experimental Example 5: Test of cell death effect test in melanoma cell line by co-administration of human PBMC activated with IL-2 and Trabedersen in combination**

Experiments were performed on the melanoma cell line (Hs 294T) in a similar manner to Experimental Example 3.

**Experimental Example 6: Test of cell death effect in lung cancer cell line by co-administration of human PBMC activated with IL-2 and Trabedersen in combination**

Experiments were performed on the lung cancer cell line (A549) in a similar manner to Experimental Example 3.

**Experimental Example 7: Test of cell death effect in colorectal cancer cell line by co-administration of human PBMC activated with IL-2 and Trabedersen in combination**

Experiments were performed on colon cancer cell lines (HT29, DLD-1, LS174T) in a similar manner to Experimental Example 3.

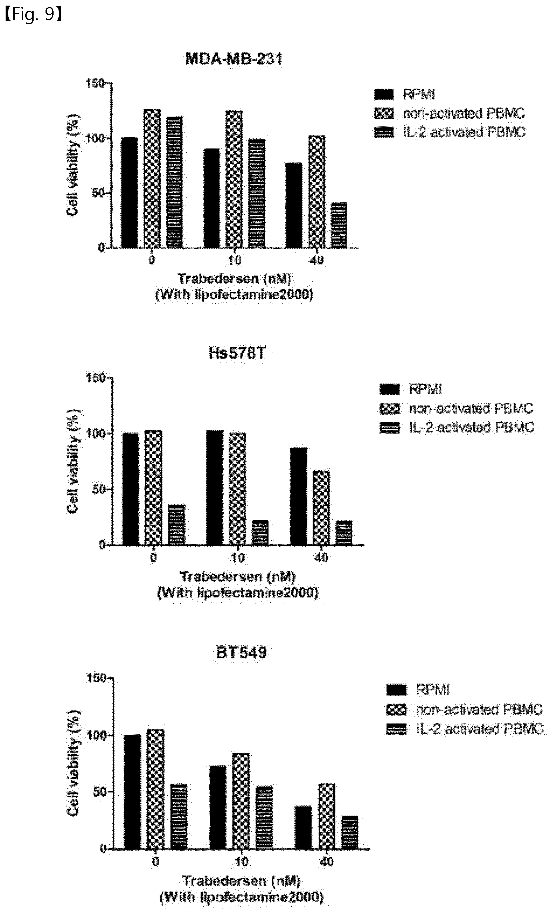

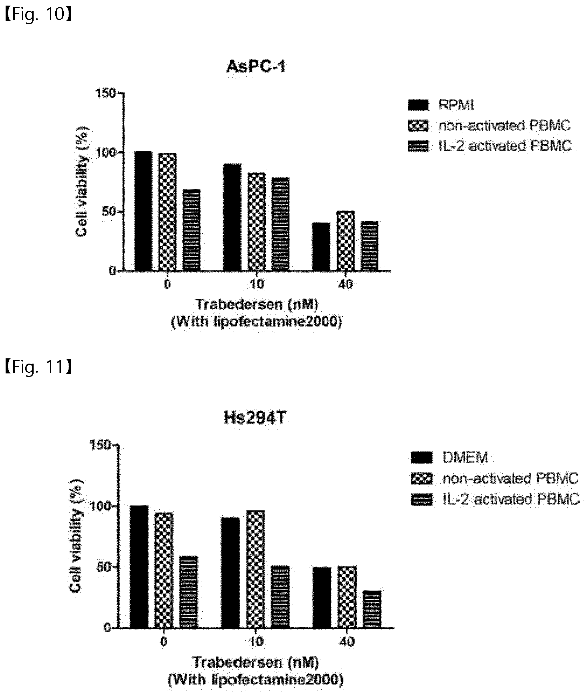

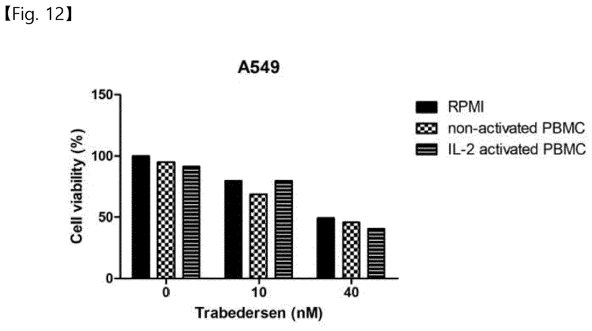

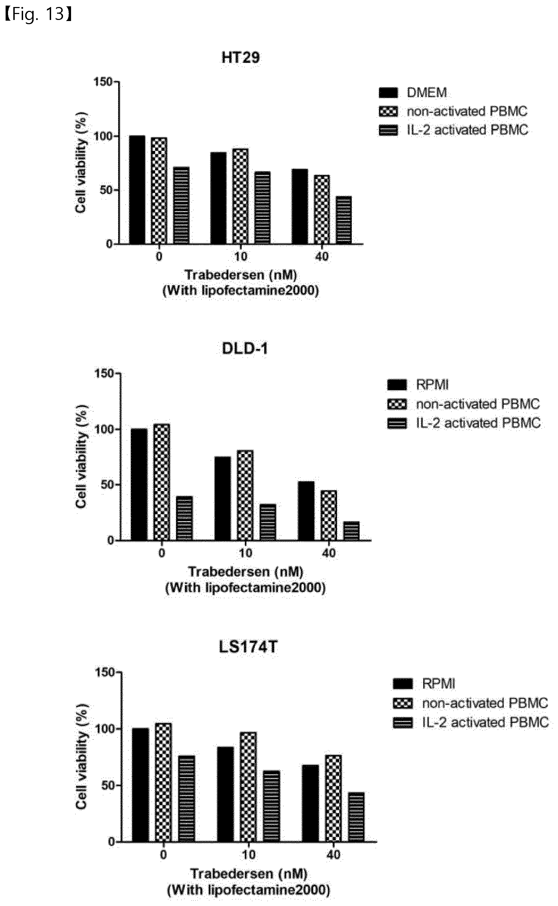

The experimental results of Experimental Examples 3 to 7 are shown in Figs. 9 to 13. As shown, it can be seen that the cell death effect of co-administration group of trabedersen and IL-2 (IL-2 activated PBMC) in combination was more significantly increased than that of the group of administration of trabedersen alone (non-activated PBMC and DMEM or RPMI) or the group of administration of IL-2 alone (IL-2 activated PBMC, trabedersen 0 nM).

**Experimental Example 8: (in vivo test) Efficacy test of tumor growth inhibition by co-administration of IL-2 and trabedersen in immunodeficient mice (NSG mice) humanized with human PBMC in triple negative breast cancer cell lines**

An experiment was conducted to determine the effects on the growth of MDA-MB-231 which is a human triple negative breast cancer (TNBC) cell line in cases of administration of trabedersen alone, administration of IL-2 alone, and co-administration of trabedersen and IL-2 in combination, respectively in mice humanized by injecting human PBMC into NSG (NOD/SCID/Gamma) immunodeficient mice

Method of experiment

Trabedersen (TBD) in the form of a lyophilized powder and recombinant human IL-2 (rhIL-2, JW Creagene, South Korea; Cat. No. JW-H031-0025) in a liquid form were prepared. The storage conditions for trabedersen are 2~8°C, and the storage conditions for recombinant human IL-2 are -80°C.

Trabedersen was dissolved in sterile physiological saline and used. For the recombinant human IL-2, a $25\mu g/250\mu\ell$ ($2\times10^8$ IU/mg) solution was diluted immediately before administration and used.

NSG mice were divided into 10 groups of 6 mice each, and they were administered intraperitoneally (IP) with the doses shown in Table 3 below.

[Table 3]

| Group | n | PBMC (Cells/mouse) | Treatment Regimen | | |
|---|---|---|---|---|---|
| | | | Agent | Dose | Route |
| 1 | 6 | 0 | TBD | 0 | IP |
| | | | IL-2 | 0 | |
| 2 | 6 | $1\times10^7$ | TBD | 0 | IP |
| | | | IL-2 | 0 | |
| 3 | 6 | $1\times10^7$ | TBD | 8 mg/kg | IP |
| | | | IL-2 | 0 | |
| 4 | 6 | $1\times10^7$ | TBD | 16 mg/kg | IP |
| | | | IL-2 | 0 | |
| 5 | 6 | $1\times10^7$ | TBD | 8 mg/kg | IP |
| | | | IL-2 | $1\times10^4$ IU | |
| 6 | 6 | $1\times10^7$ | TBD | 8 mg/kg | IP |
| | | | IL-2 | $5\times10^4$ IU | |
| 7 | 6 | $1\times10^7$ | TBD | 16 mg/kg | IP |
| | | | IL-2 | $1\times10^4$ IU | |
| 8 | 6 | $1\times10^7$ | TBD | 16 mg/kg | IP |
| | | | IL-2 | $5\times10^4$ IU | |

(continued)

| Group | n | PBMC | Treatment Regimen | | |
|---|---|---|---|---|---|
| | | (Cells/mouse) | Agent | Dose | Route |
| 9 | 6 | $1 \times 10^7$ | TBD | 0 mg/kg | IP |
| | | | IL-2 | 1×10^4 IU | |
| 10 | 6 | $1 \times 10^7$ | TBD | 0 mg/kg | IP |
| | | | IL-2 | 5×10^4 IU | |

Xenograft model preparation

MDA-MB-231 cells are grown in a culture environment of 37°C 5% $CO_2$. RPMI 1640 (including L-glutamine, 25mM HEPES) media containing 10% FBS was used. When cells occupied 70-80% of the surface of the 25T flask, they were subcultured at a ratio of 1:5.

On the day of tumor inoculation, the cultured tumor cells were suspended in PBS at $1 \times 10^7$ cells/100$\mu\ell$, and each 100$\mu\ell$ was implanted subcutaneously into the right flank of the mouse so as to be $1 \times 10^7$ cells/mouse.

Human-PBMC (Hu-PBMC) NSG mouse model preparation

Human PBMCs were thawed in a 37°C water bath, washed twice with RPMI 1640 (including DNAase I) containing 20% FBS, and then stabilized for 30 minutes under culture environment of 37°C 5% $CO_2$.

After 30 minutes of incubation, PBMCs were diluted in PBS to a concentration of $5 \times 10^7$ to $10 \times 10^7$ cells/m$\ell$, and then administered at a dose of $1 \times 10^7$ cells/head per mouse (PBMCs were administered on the 3$^{rd}$ day after cancer cell transplantation).

Method of Administration

To the mice which are administered with PBMC as described above, trabedersen was administered intraperitoneally on the 2$^{nd}$ day after PBMC administration (*i.e.,* on the 5$^{th}$ day after the cancer cell transplantation) at a dose of Table 3 for a total of 8 times every other day (once/2 days), and then IL-2 was administered on the 4$^{th}$ day after PBMC administration (i.e., on the 7$^{th}$ day after tumor cell transplantation) as a dose of Table 3 for 4 days (once/day), and then no administration period of 3 days. This is one cycle schedule and a total of two cycles for intraperitoneal administration are conducted.

The schedules for inoculation, PBMC administration, trabedersen administration, and IL-2 administration are as follows:

| Day 0 | Day 3 | Day 5 | Day 7 |
|---|---|---|---|
| Tumor inoculation | PBMC administration | Starting of TBD administration | Starting of IL-2 adminstration |

Tumor Volume Measurement

After administration of trabedersen and/or IL-2, the tumor volume was measured every other day (once/2 days), i.e., the tumor volume was measured every other day from the 5$^{th}$ day after tumor inoculation (Day 0). The tumor volume was calculated from the following equation by measuring the size of the tumor mass in the injected region with a caliper (CD-15CPX, digimatic caliper, Mitutoyo, Japan):

[Equation]

$$\text{Tumor volume (TV; mm}^3) = (W^2 \times L)/2$$

wherein W is the length of short axis and L is the length of long axis.

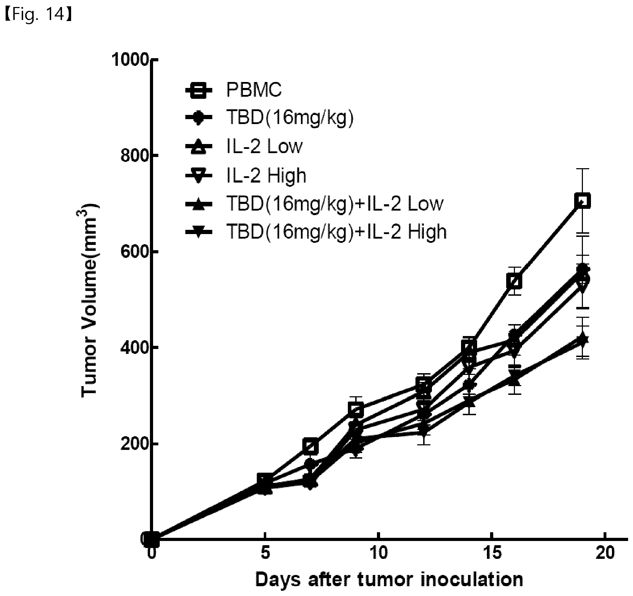

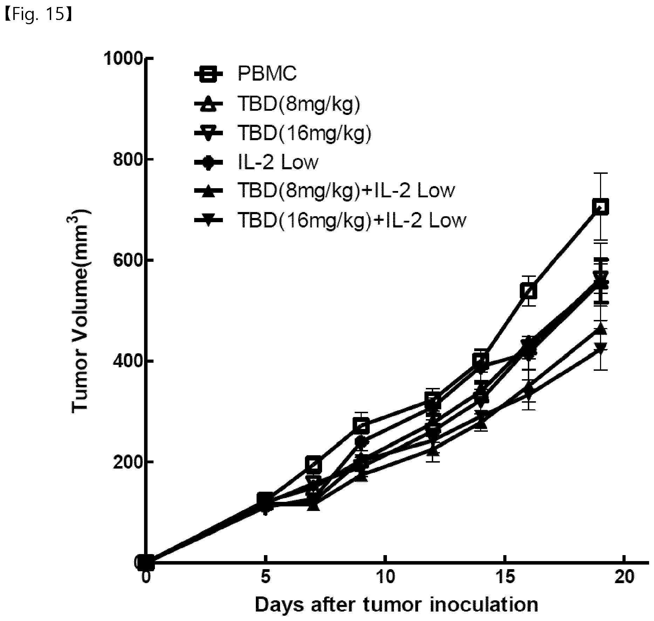

According to the result of the above experiment, it can be seen that the effect of inhibiting tumor growth is exerted synergistically when trabedersen and IL-2 are administered in combination in comparison with the case where trabedersen is administered alone or where IL-2 is administered alone (see Figs. 14 and 15).

[Industrial applicability]

The composition of combination according to the present invention can be used for anticancer treatment.

**Claims**

1.  A composition of combination for treating cancer comprising trabedersen and IL-2.

2.  The composition of combination for treating cancer according to Claim 1, wherein the Trabedersen and IL-2 are to be administered simultaneously, sequentially, or separately.

3.  The composition of combination for treating cancer according to Claim 1, wherein the composition is for treating solid cancer.

4.  The composition of combination for treating cancer according to Claim 3, wherein the solid cancer is breast cancer, pancreatic cancer, melanoma, lung cancer, or colorectal cancer.

5.  A composition for treating cancer comprising trabedersen for administration in combination with IL-2.

6.  The composition for treating cancer according to Claim 5, wherein the composition is for treating solid cancer.

7.  The composition for treating cancer according to Claim 6, wherein the solid cancer is breast cancer, pancreatic cancer, melanoma, lung cancer, or colorectal cancer.

8.  A composition for treating cancer comprising IL-2 for administration in combination with trabedersen.

9.  The composition for treating cancer according to Claim 8, wherein the composition is for treating solid cancer.

10. The composition for treating cancer according to Claim 9, wherein the solid cancer is breast cancer, pancreatic cancer, melanoma, lung cancer, or colorectal cancer.

【Fig. 1】

【Fig. 2】

【Fig. 3】

【Fig. 4】

**MDA-MB-231**

**Hs578T**

**BT549**

【Fig. 5】

AsPC-1

Capan-2

【Fig. 6】

【Fig. 7】

【Fig. 8】

【Fig. 9】

【Fig. 10】

**AsPC-1**

【Fig. 11】

**Hs294T**

【Fig. 12】

**A549**

【Fig. 13】

【Fig. 14】

【Fig. 15】

## INTERNATIONAL SEARCH REPORT

| International application No. |
| --- |
| PCT/KR2019/012178 |

| A. CLASSIFICATION OF SUBJECT MATTER |
| --- |
| *A61K 31/7088(2006.01)i, A61K 38/20(2006.01)i, A61P 35/00(2006.01)i* |
| According to International Patent Classification (IPC) or to both national classification and IPC |

| B. FIELDS SEARCHED |
| --- |
| Minimum documentation searched (classification system followed by classification symbols) <br> A61K 31/7088; A61K 31/18; A61K 31/435; A61K 38/19; A61N 1/39; A61K 38/20; A61P 35/00 |
| Documentation searched other than minimum documentation to the extent that such documents are included in the fields searched <br> Korean utility models and applications for utility models: IPC as above <br> Japanese utility models and applications for utility models: IPC as above |
| Electronic data base consulted during the international search (name of data base and, where practicable, search terms used) <br> eKOMPASS (KIPO internal) & Keywords: anticancer, trabedersen, IL-2(interlukin-2), TGF-b2 inhibitor, combination, co-administration |

| C. DOCUMENTS CONSIDERED TO BE RELEVANT | | |
| --- | --- | --- |
| Category* | Citation of document, with indication, where appropriate, of the relevant passages | Relevant to claim No. |
| Y | PARK, J. et al. Combination delivery of TGF-β inhibitor and IL-2 by nanoscale liposomal polymeric gels enhances tumour immunotherapy. Nature materials. 2012, vol. 11, no. 10, pages 895-905 <br> See abstract; page 902, right column-page 903, left column. | 1-10 |
| Y | SCHLINGENSIEPEN, K.-H. et al. Transforming growth factor-beta 2 gene silencing with trabedersen (AP 12009) in pancreatic cancer. Cancer science. June 2011, vol. 102, no. 6, pages 1193-1200 <br> See abstract; page 1197. | 1-10 |
| Y | ALVAREZ, M. et al. Increased antitumor effects using IL-2 with anti -TGF-β reveals competition between mouse NK and CD8 T cells. The Journal of Immunology. 2014 (Electronic publication 07 June 2014 ), vol. 193, pages 1709-1716 <br> See abstract; pages 1713-1714. | 1-10 |
| Y | WO 2018-045346 A1 (AUTOTELIC LLC.) 08 March 2018 <br> See abstract; claims 1-33. | 1-10 |
| A | US 2007-0184020 A1 (KALBE, J. et al.) 09 August 2007 <br> See the entire document. | 1-10 |

| ☒ Further documents are listed in the continuation of Box C. | ☒ See patent family annex. |
| --- | --- |

| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| --- | --- |
| Date of the actual completion of the international search <br><br> 27 DECEMBER 2019 (27.12.2019) | Date of mailing of the international search report <br><br> **30 DECEMBER 2019 (30.12.2019)** |
| Name and mailing address of the ISA/KR <br> Korean Intellectual Property Office <br> Government Complex Daejeon Building 4, 189, Cheongsa-ro, Seo-gu, Daejeon, 35208, Republic of Korea <br> Facsimile No. +82-42-481-8578 | Authorized officer <br><br><br> Telephone No. |

Form PCT/ISA/210 (second sheet) (January 2015)

**INTERNATIONAL SEARCH REPORT**

| International application No. |
| --- |
| **PCT/KR2019/012178** |

| C (Continuation). | DOCUMENTS CONSIDERED TO BE RELEVANT | | |
| --- | --- | --- |
| Category* | Citation of document, with indication, where appropriate, of the relevant passages | Relevant to claim No. |
| A | WO 2017-218544 A1 (CHEMOCENTRYX, INC.) 21 December 2017<br>See the entire document. | 1-10 |

Form PCT/ISA/210 (continuation of second sheet) (January 2015)

**INTERNATIONAL SEARCH REPORT**
Information on patent family members

International application No.

**PCT/KR2019/012178**

| Patent document cited in search report | Publication date | Patent family member | Publication date |
|---|---|---|---|
| WO 2018-045346 A1 | 08/03/2018 | None | |
| US 2007-0184020 A1 | 09/08/2007 | CA 2567477 A1 | 22/12/2005 |
| | | CN 1984694 A | 20/06/2007 |
| | | DE 102004028156 A1 | 05/01/2006 |
| | | EP 1753507 A1 | 21/02/2007 |
| | | JP 2008-537489 A | 18/09/2008 |
| | | WO 2005-120638 A1 | 22/12/2005 |
| | | WO 2005-120638 A8 | 18/01/2007 |
| WO 2017-218544 A1 | 21/12/2017 | AU 2017-283491 A1 | 20/12/2018 |
| | | BR 112018075660 A2 | 09/04/2019 |
| | | CA 3026515 A1 | 21/12/2017 |
| | | CN 109562086 A | 02/04/2019 |
| | | EP 3468548 A1 | 17/04/2019 |
| | | JP 2019-518038 A | 27/06/2019 |
| | | KR 10-2019-0017913 A | 20/02/2019 |
| | | MX 2018015172 A | 04/07/2019 |
| | | SG 11201810940 A | 30/01/2019 |
| | | US 10195188 B2 | 05/02/2019 |
| | | US 10251888 B2 | 09/04/2019 |
| | | US 10398685 B2 | 03/09/2019 |
| | | US 2017-0354657 A1 | 14/12/2017 |
| | | US 2017-0368043 A1 | 28/12/2017 |
| | | US 2019-0167650 A1 | 06/06/2019 |
| | | US 2019-0350911 A1 | 21/11/2019 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHLINGENSIEPEN KH et al.** *Cancer Sci.,* 2011, vol. 102, 1193-200